# NEW EUROPEAN PATENT SPECIFICATION

(11) **EP 1 345 637 B2**
(45) Date of publication and mention of the opposition decision: **08.07.2015**
(45) Mention of the grant of the patent: 02.03.2011
(21) Application number: 01272430.8
(22) Date of filing: 21.12.2001
(51) Int. Cl.: A61L 27/06

(54) **SURFACE MODIFICATION OF IMPLANTS FOR HEALING IN BONE AND SOFT TISSUE**
OBERFLÄCHENMODIFIZIERUNG VON IMPLANTATEN ZUR HEILUNG IN KNOCHEN UND GEWEBEN
MODIFICATION DE SURFACE D'IMPLANTS UTILISES DANS LA CICATRISATION DE TISSUS OSSEUX ET DE TISSUS MOUS

(30) Priority: 22.12.2000 SE 0004854
(43) Date of publication of application: 24.09.2003
(73) Proprietor: TioTec AB, 540 16 Timmersdala (SE)
(72) Inventor: Nygren, Hakan, 427 38 Billdal (SE)
(74) Representative: Romare, Laila Anette
(86) International application number: PCT/SE2001/002874
(87) International publication number: WO 2002/051465

(56) References cited:
- WO-A1-00/72776
- WO-A1-00/72777
- US-A- 5 700 559
- US-A- 5 935 594
- US-B2- 7 393 391
- NYGREN, H.: "Initial reactions of whole blood with hydrophilic and hydrophobic titanium surfaces" COLLOIDS AND SURFACES B: BIOINTERFACES, vol. 6, 1996, pages 329-333,
- ZREIQAT H. ET AL: 'Effect of surface chemical modification of bioceramic on phenotype of human bone-derived cells' JOURNAL OF BIOMEDICAL MATERIALS RESEARCH vol. 44, no. 4, 15 March 1999, pages 389 - 396

## Description

### TECHNICAL FIELD

The application relates to an implant intended for incorporation in bone and soft tissue and comprising a metal or a semiconductor and a layer of a metal oxide or a semiconductor oxide. The application also relates to a method for treating the surface of said implant.

### PRIOR ART

On implantation of foreign material into the body tissues, an inflammatory reaction always occurs which can either be part of the incorporation process, and thus entirely necessary for a successful result, or part of a rejection reaction or fibrous encapsulation of the material, which results in clinical failure.

Various studies have been made of the mechanisms which take place upon implantation of foreign material into the body tissues. Cell death and downward regulation of macrophage functions are described by Nygren et al. (J. Biomed. Mater. Res. 45, 117-124, 1999).

Examples of materials which are used in implants are silicon/quartz, aluminium/aluminium oxide and titanium/titanium oxide. Of these, titanium/titanium oxide has previously been used with great success as implantation material (Leventhal, G. S., Bone Joint Surg., 33A, 473-474, 1951).

It has already been found to be positive if the material which is implanted has pores. The advantageous effect of pores in that part of the material in contact with tissue has been described by a number of researchers all concerned with the phenomenon of "foreign-body tumours". It is known that tight membranes of cellulose or silicone rubber give rise to tumours in rats more than a year after implantation. By providing pores with a diameter of at least 0.65 µm, it was possible to avoid this tumour-inducing effect of the membrane (James et al., Biomaterials 18, 1997, 667-675).

However, despite extensive research in this field, there are still problems in improving the incorporation of implants in bone and soft tissue.

WO 00/72776 A1 and WO 00/72777 A1 disclose implants intended for incorporation in bone and soft tissue comprising titanium and a porous surface layer of titanium oxide. The pores may contain a bone-growth-initiating organic substance.

### DISCLOSURE OF THE INVENTION

According to the invention, the above problems are solved by constructing an implant which comprises a metal or a semiconductor and a surface layer of metal oxide or semiconductor oxide on the surface of the metal or the semiconductor. The oxide layer is hydrophobic and has pores. Additionally, the oxyde layer is coated with a layer of magnesium. It has surprisingly been found that a synergistic effect is achieved by combining an oxide layer with the above two first mentioned properties. According to the invention, a method for treating the surface of an implant according to the above comprises the steps of making the oxide porous and of making the oxide hydrophobic and of coating the oxide layer with a layer of magnesium.

By studies of the early inflammatory reaction around the implant, and by means of subsequent work on the invention, it has been possible, according to the invention, to find a way of reducing the intensity of the initial inflammation, measured as cell death and downward regulation of macrophage functions.

By means of these studies, it has been discovered that this cell death and downward regulation of macrophage functions reflects the capacity for incorporation of the material such that increased cell death upon material contact is a negative property. By means of systematic studies of which material properties influence incorporation, it is possible to distinguish three important parameters, of which two are essential to the invention, and of which the third affords a further advantage.

According to a preferred embodiment, the pores in the oxide layer have a pore size in the range of 0.2 µm - 3.0 µm, preferably in the range of 0.2 µm - 1.0 µm. The pore size can be measured, for example, with the aid of an electron microscope, the lower limit of the range being defined as the minimum diametrical cross section across the pore, and the upper limit of the range being defined as the maximum diametrical cross section across the pore.

One feature which determines what pore size is most suitable is the draining effect which the pores have on undesired substances from the area of incorporation. The lower limit for this effect is ca. 0.2 µm. The pore size is also dependent on the size of the cells in the implantation area. Cells must not enter the pores.

There are various ways of making the oxide porous. According to one embodiment, the oxide is made porous by anodization in an electrolyte consisting of 0.1 to 1% by volume of hydrofluoric acid. According to an alternative embodiment, the oxide is made porous by anodization in an electrolyte consisting of 0.1 to 1 % by volume of hydrofluoric acid and 10% by volume of nitric acid.

According to one embodiment, the oxide layer is made hydrophobic by incubating the layer in an atmosphere of hexamethyl disilazane. Other substances can of course be used for the alkylation. Within the scope of the invention, the oxide layer can be made hydrophobic in ways other than by alkylation.

According to a preferred embodiment of the invention, the oxide layer has a layer of a substance which neutralizes oxygen radicals. This substance can be glutathione, for example. It has previously been found that a favourable effect is achieved by means of covalent bonding of glutathione to gold surfaces (Nygren et al., J. Biomed. Mater. Res. 45, 117-124, 1999). The arrangement proposed here differs from published data in that a porous oxide is used as substrate and in that it is hydrophobic. According to another preferred embodiment of the invention, it is possible to avoid coupling glutathione covalently to the material, and instead, according to this embodiment, a surface layer of substances which neutralize oxygen radicals is adsorbed or absorbed and evaporated onto the surface of the oxide.

The implant can consist of one material or can include different materials. It can be a metal such as titanium, or a semiconductor such as silicon. According to a preferred embodiment, the implant consists of titanium with a surface layer of titanium dioxide.

In the case of implantation in bone, the later phase of development can be favourably influenced by coating with a layer of magnesium. Early studies of pure magnesium implants revealed increased callus formation in connection with the metal's dissolution and absorption in the body (E. D. McBride, Southern Medical Journal 31: (5) 508, 1938). Later studies with ion-implanted magnesium in metal oxides show that small amounts of magnesium stimulate differentiation of bone-forming cells in contact with the material (Zreiqat et al., J. Biomed. Mater. Res. 44, 389, 1999). The capacity for incorporation achieved with an arrangement according to the invention includes that the oxide layer being coated with a layer of magnesium. According to a preferred embodiment, the magnesium is applied electrolytically, using the implant as cathode. This method is not already known.

By means of the invention, it has been found that hydrophobicization, such as alkylation of metal surfaces or semiconductor surfaces, such as titanium surfaces, so that these obtain a hydrophobic surface, is favourable with regard to the survival of macrophages in contact with the material. The conclusion therefore is that an implant should be produced which comprises a metal oxide or semiconductor oxide with pores, preferably alkylated in order to obtain a hydrophobic surface and preferably provided with a layer of substance which neutralizes oxygen radicals. Ways of achieving each of these properties are described in the examples below. In summary, the invention affords an unexpected and advantageous effect, and an arrangement not previously described for implantation in bone and soft tissue.

According to the invention, the surface modification of the implant can be carried out using the following techniques:

### 1. Porous metal oxides and semiconductor oxides.

Porous metal oxides or semiconductor oxides are generally produced by two competing processes, etching of the oxide and oxide growth are allowed to act simultaneously. For example, it is known to etch silicon dioxide with hydrofluoric acid at the same time as new oxide is anodized on the material (Professor Hans Arwin IFM, LiT H, personal communication). A corresponding technique has been described for aluminium oxide which can be made porous by anodization in sulphuric acid (Gonzalez et al., J. Electrochem. Soc. 147, 984, 2000). Titanium dioxide is soluble in hydrofluoric acid (Merck Index) and can be provided with a porous oxide layer in a corresponding manner.

### 2. Alkylation of metal oxides and semiconductor oxides.

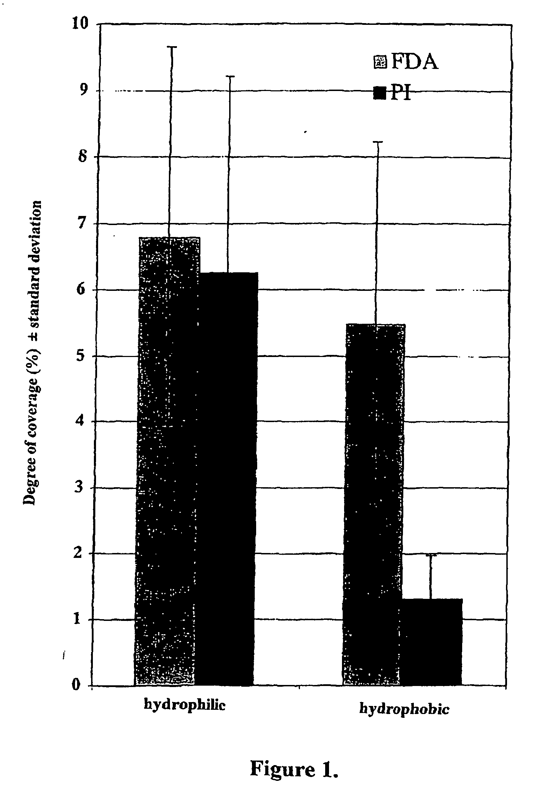

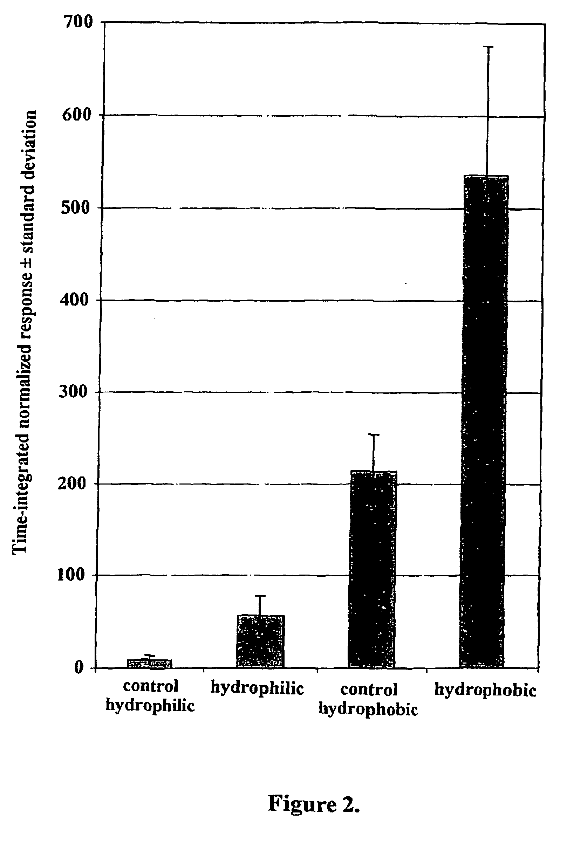

A hydrophobic surface can be obtained by alkylation of metal oxides and semiconductor oxides. Titanium oxide can be made hydrophobic by incubation in butanol (Nygren, H. Colloids and Surfaces B: Biointerfaces 6, 329, 1996) or by treatment with hexamethyl disilazane in gaseous phase or dissolved in suitable solvent. The latter method can also be used for alkylation of silicon oxide and aluminium oxide. The result is that the surface is covered with methyl groups (-CH₃) and is hydrophobic. Titanium oxide with a hydrophobic surface has been found to have a less downwardly regulating effect on cells in contact with the implant than do corresponding hydrophilic materials (Figure 2). A higher proportion of cells also survive in contact with the material (Figure 1).

### 3. Addition of substances which neutralize oxygen radicals.

Substances which neutralize oxygen radicals can be substances which have a direct neutralizing action and are themselves destroyed in the reaction with oxygen radicals, for example tocopherol, glutathione and cysteine. Enzymes such as superoxide dismutase and catalase break down oxygen radicals to hydrogen peroxide and water and are not affected themselves by the reaction. All these different types of substances which neutralize oxygen radicals have been used in different contexts for acting on inflammation processes, and the use of these substances is known in the art. Glutathione has been coupled covalently to gold surfaces (Lestelius et al. J. Biomed. Mater. Res. 28 871, 1994) and in such cases has been shown to have an unexpected and favourable effect on cell survival in contact with implants (Nygren et al. J. Biomed. Mater. Res. 45, 117-124, 1999).

### 4. Inclusion of magnesium in metal oxides

Implantation of magnesium in aluminium oxide for the purpose of stimulating bone formation has previously been done using ion implant techniques (Zreiqat et al., J. Biomed. Mater. Res. 44, 389, 1999). This is a possible way of applying magnesium in accordance with the invention.

### BRIEF DESCRIPTION OF THE FIGURES

Figure 1 shows that cell survival (FDA⁺) is higher on an alkylated titanium oxide surface.
Figure 2 shows that the stimulation capacity for PMA (phorbol myristate acetate) is better on an alkylated titanium oxide surface.
Figure 3 shows a scanning electron microscope image of porous titanium oxide which has been produced by anodization in 0.5% by volume of HF, at 50V for 90 minutes.
Figure 4 shows a scanning electron microscope image of porous titanium oxide which has been produced by anodization in 0.5% by volume of HF and 10% by volume of HNO₃ at 25V for 30 minutes.

### ILLUSTRATIVE EMBODIMENTS

### Production of a porous surface layer of TiO₂ on titanium

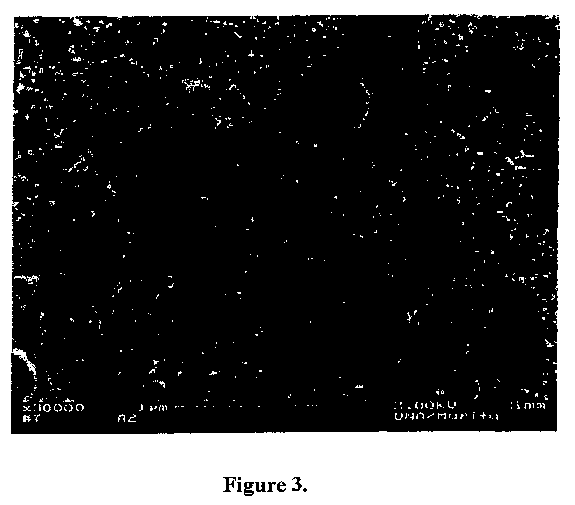

Pieces (8 x 8 x 1 mm) of commercially available titanium plate were mounted in a section of titanium and coupled as anode in an electrophoresis apparatus. The cathode was made of platinum wire, and the electrolyte used was hydrofluoric acid (HF) in a concentration of 0.5% by volume. A voltage of 50V was applied between the electrodes and a current of 2 mA was read off at the voltage sensor. The result after 90 minutes of anodization in 0.5% by volume of HF is shown in Figure 3.

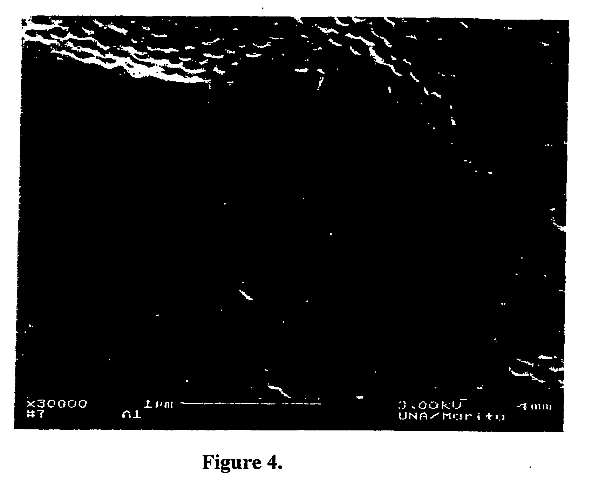

A better result can be obtained by increasing the rate of oxidation by addition of a further acid. Electrolysis was carried out with a mixture of HF (0.5% by volume) and HNO₃ (10% by volume) as electrolyte. After 30 minutes at a voltage of 25V, the result shown in Figure 4 was obtained.

### Production of a hydrophobic porous surface layer of TiO₂ on titanium

The pieces were made hydrophobic by placing them in a saturated atmosphere of hexamethyl disilazane, or by washing them in a mixture of 90% by volume of butanol and 10% by volume of water.

### Production of an oxygen-radical-neutralizing hydrophobic porous surface layer of TiO₂ on titanium

The hydrophobic pieces were immersed in an aqueous solution of 100 um of glutathione or an alcohol solution of 100 µglml of tocopherol, or a mixture of superoxide bismuthase (10 µg/ml) and catalase (90 µglml) and allowed to dry.

### Inclusion of magnesium in the implant surface

Porous titanium was placed as cathode in an electrolysis bath with 0.1 M MgCl₂ and platinum as anode. At a voltage of 50V, a layer of magnesium is formed at the bottom of the pores after 10 minutes of electrolysis.

## Claims

1. Implant intended for incorporation in bone and soft tissue and comprising a metal or a semiconductor and a layer of a metal oxide or a semiconductor oxide on the surface of the metal or the semiconductor, where the oxide layer is hydrophobic and has pores, and where the oxide layer is coated with a layer of magnesium.

2. Implant according to Claim 1, where the pores in the oxide layer have a minimum diameter of 0.2 µm.

3. Implant according to Claim 1 or 2, where the pores in the oxide layer have a maximum diameter of 3.0 µm.

4. Implant according to Claim 1 or 2, where the pores in the oxide layer have a maximum diameter of 1.0 µm.

5. Implant according to any of the preceding claims, where the surface of the oxide layer has a layer of substance which neutralizes oxygen radicals.

6. Implant according to Claim 5, where the substance which neutralizes oxygen radicals is glutathione.

7. Implant for implantation in bone or soft tissue according to any of the preceding claims, where the metal is titanium and the oxide is titanium dioxide.

8. Method for treating the surface of an implant intended for implantation in bone or soft tissue and made of a metal or semiconductor which on the surface has a layer of metal oxide and/or semiconductor oxide, said method comprising the steps of: the oxide layer being made porous, the oxide layer being made hydrophobic, and the oxide layer is coated with a layer of magnesium.

9. Method for treating the surface of an implant for implantation in bone or soft tissue according to Claim 8, comprising a step in which the oxide layer is treated with a substance which neutralizes oxygen radicals.

10. Method for treating the surface of an implant for implantation in bone or soft tissue according to Claim 9, comprising a step in which the substance which neutralizes oxygen radicals is bound to the oxide layer by adsorption or absorption.

11. Method for treating the surface of an implant for implantation in bone or soft tissue according to Claim 9 or 10, in which the substance which neutralizes oxygen radicals is glutathione.

12. Method for treating the surface of an implant for bone or soft tissue according to any of Claims 8 to 11, in which the oxide is made porous by anodization in an electrolyte consisting of 0.5-0.1% by volume of hydrofluoric acid.

13. Method for treating the surface of an implant for bone or soft tissue according to one of Claims 8 to 11, in which the oxide is made porous by anodization in an electrolyte consisting of 0.5-0.1% by volume of hydrofluoric acid and 10% by volume of nitric acid.

14. Method according to any of Claims 8 to 13, in which the oxide layer is made hydrophobic by incubating the layer in an atmosphere of hexamethyl disilazane.

15. Method for treating the surface of an implant for implantation in bone or soft tissue according to any of Claims 8 to 14, in which the magnesium is applied electrolytically, using the implant as cathode.

## Patentansprüche

1. Implantat, das zum Einfügen in Knochen und weiches Gewebe vorgesehen ist und das ein Metall oder einen Halbleiter und eine Schicht aus einem Metalloxid oder einem Halbleiteroxid auf der Oberfläche des Metalls oder des Halbleiters umfasst, wobei die Oxidschicht hydrophob ist und Poren aufweist, und wobei die Oxidschicht mit einer Magnesiumschicht beschichtet ist.

2. Implantat gemäss Anspruch 1, wobei die Poren in der Oxidschicht einen Mindestdurchmesser von 0,2 µm haben.

3. Implantat gemäss Anspruch 1 oder 2, wobei die Poren in der Oxidschicht einen Maximaldurchmesser von 3,0 µm haben.

4. Implantat gemäss Anspruch 1 oder 2, wobei die Poren in der Oxidschicht einen Maximaldurchmesser von 1,0 µm haben.

5. Implantat gemäss einem der vorangehenden Ansprüche, wobei die Oberfläche der Oxidschicht eine Substanzschicht aufweist, die Sauerstoffradikale neutralisiert.

6. Implantat gemäss Anspruch 5, wobei die Substanz, die Sauerstoffradikale neutralisiert, Glutathion ist.

7. Implantat zum Implantieren in Knochen oder weiches Gewebe gemäss einem der vorangehenden Ansprüche, wobei das Metall Titan ist und das Oxid Titandioxid ist.

8. Verfahren zur Behandlung der Oberfläche eines Implantats, das zum Implantieren in Knochen oder weiches Gewebe vorgesehen ist und das aus einem Metall oder einem Halbleiter, der an der Oberfläche eine Schicht von einem Metalloxid und/oder einem Halbleiteroxid aufweist, hergestellt ist, wobei das Verfahren die folgenden Schritte umfasst: die Oxidschicht wird porös gemacht, die Oxidschicht wird hydrophob gemacht, und die Oxidschicht wird mit einer Magnesiumschicht beschichtet.

9. Verfahren zur Behandlung der Oberfläche eines Implantats zum Implantieren in Knochen oder weiches Gewebe gemäss Anspruch 8, wobei das Verfahren einen Schritt umfasst, in dem die Oxidschicht mit einer Substanz, die Sauerstoffradikale neutralisiert, behandelt wird.

10. Verfahren zur Behandlung der Oberfläche eines Implantats zum Implantieren in Knochen oder weiches Gewebe gemäss Anspruch 9, wobei das Verfahren einen Schritt umfasst, in dem die Substanz, welche Sauerstoffradikale neutralisiert, durch Adsorption oder Absorption an die Oxidschicht gebunden wird.

11. Verfahren zur Behandlung der Oberfläche eines Implantats zum Implantieren in Knochen oder weiches Gewebe gemäss den Ansprüchen 9 oder 10, wobei die Substanz, welche Sauerstoffradikale neutralisiert, Glutathion ist.

12. Verfahren zur Behandlung der Oberfläche eines Implantats für Knochen oder weiches Gewebe gemäss den Ansprüchen 8 bis 11, wobei das Oxid durch Eloxieren in einem Elektrolyten, der aus 0,5 bis 0,1 Vol.% Fluorwasserstoffsäure besteht, porös gemacht wird.

13. Verfahren zur Behandlung der Oberfläche eines Implantats für Knochen oder weiches Gewebe gemäss den Ansprüchen 8 bis 11, wobei das Oxid durch Eloxieren in einem Elektrolyten, der aus 0,5 bis 0,1 Vol.% Fluorwasserstoffsäure und 10 Vol.% Salpetersäure besteht, porös gemacht wird.

14. Verfahren gemäss den Ansprüchen 8 bis 13, wobei die Oxidschicht durch Aussetzen der Schicht einer Atmosphäre von Hexamethyldisilazan hydrophob gemacht wird.

15. Verfahren zur Behandlung der Oberfläche eines Implantats zum Implantieren in Knochen oder weiches Gewebe gemäss den Ansprüchen 8 bis 14, wobei das Magnesium elektrolytisch aufgetragen wird, indem das Implantat als Kathode verwendet wird.

## Revendications

1. Implant destiné à être incorporé dans l'os et le tissu mou et comprenant un métal ou un semi-conducteur et une couche d'un oxyde métallique ou d'un oxyde de semi-conducteur sur la surface du métal ou du semi-conducteur, où la couche d'oxyde est hydrophobe et a des pores, dans lequel la couche d'oxyde est revêtue d'une couche de magnésium.

2. Implant selon la revendication 1, dans lequel les pores dans la couche d'oxyde ont un diamètre minimum de 0,2 µm.

3. Implant selon les revendications 1 ou 2, dans lequel les pores dans la couche d'oxyde ont un diamètre maximum de 3,0 µm.

4. Implant selon les revendications 1 ou 2, dans lequel les pores dans la couche d'oxyde ont un diamètre maximum de 1,0 µm.

5. Implant selon l'une quelconque des revendications précédentes, dans lequel la surface de la couche d'oxyde a une couche de substance qui neutralise les radicaux d'oxygène.

6. Implant selon la revendication 5, dans lequel la substance qui neutralise les radicaux d'oxygène est la glutathione.

7. Implant pour implantation dans l'os ou le tissu mou, selon l'une quelconque des revendications précédentes, dans lequel le métal est le titane et l'oxyde est le dioxyde de titane.

8. Méthode de traitement de la surface d'un implant destiné à l'implantation dans un os ou un tissu mou et constitué d'un métal ou d'un semi-conducteur qui, sur la surface, a une couche d'oxyde métallique et/ou d'oxyde de semi-conducteur, ladite méthode comprenant les étapes consistant à : rendre la couche d'oxyde poreuse, rendre la couche d'oxyde hydrophobe, et la couche d'oxyde est revêtue d'une couche de magnésium.

9. Méthode de traitement de la surface d'un implant pour implantation dans un os ou un tissu mou selon la revendication 8, comprenant une étape, dans laquelle la couche d'oxyde est traitée avec une substance qui neutralise les radicaux d'oxygène.

10. Méthode de traitement de la surface d'un implant pour implantation dans un os ou un tissu mou selon la revendication 9, comprenant une étape dans laquelle la substance qui neutralise les radicaux d'oxygène est liée à la couche d'oxyde par adsorption ou absorption.

11. Méthode de traitement de la surface d'un implant pour implantation dans un os ou un tissu mou selon les revendications 9 ou 10, dans laquelle la substance qui neutralise les radicaux d'oxygène est le glutathione.

12. Méthode de traitement de la surface d'un implant pour un os ou un tissu mou selon l'une quelconque des revendications 8 à 11, dans laquelle l'oxyde est rendu poreux par anodisation dans un électrolyte constitué de 0,5-0,1% en volume d'acide fluorhydrique.

13. Méthode de traitement de la surface d'un implant pour un os ou un tissu mou selon l'une quelconque des revendications 8 à 11, dans laquelle l'oxyde est rendu poreux par anodisation dans un électrolyte constitué de 0,5 - 0,1% en volume d'acide fluorhydrique et de 10% en volume d'acide nitrique.

14. Méthode selon l'une quelconque des revendications 8 à 13, dans laquelle la couche d'oxyde est rendue hydrophobe en incubant la couche dans une atmosphère d'hexaméthyl-disilazane.

15. Méthode de traitement de la surface d'un implant pour implantation dans un os ou un tissu mou selon l'une quelconque des revendications 8 à 14, dans laquelle le magnésium est appliqué de manière électrolytique, en utilisant l'implant comme cathode.
